# EUROPEAN PATENT APPLICATION

(11) **EP 2 455 022 A1**
(43) Date of publication of application: **23.05.2012**
(21) Application number: 11250780.1
(22) Date of filing: 09.09.2011
(51) Int. Cl.: A61B 17/34

(54) **Adjustable surgical portal**

(30) Priority: 23.11.2010 US 416551 P; 25.08.2011 US 217717
(71) Applicant: Tyco Healthcare Group LP, New Haven, CT 06511 (US)
(72) Inventor: Stopek, Joshua, Guilford, CT 06437 (US); Sargeant, Timothy, Guildford, CT 06437 (US)
(74) Representative: Soames, Candida Jane

(57) **Abstract**

A surgical portal (100) includes a compressible, e.g., foam or rubber, portal having one or more longitudinal ports (118) for passage of a surgical object. The portal is adapted to transition between a first condition having a first length and a second condition having a second length different from the first length. The portal includes an outer wall (110) configured to maintain a substantial sealing relationship with tissue upon insertion therein.

## Description

### CROSS REFERENCE TO RELATED APPLICATION

The present application claims the benefit of and priority to U.S. Provisional Application Serial No. 61/416,551 filed on November 23, 2010, the entire contents of which are incorporated herein by reference.

### BACKGROUND

### Technical Field

The present disclosure relates generally to surgical portals for use in minimally invasive surgical procedures, such as endoscopic and/or laparoscopic procedures, and more particularly, relates to an adjustable surgical portal for inserting into a tissue tract of a patient.

### Description of Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic." Throughout the present disclosure, the term "minimally invasive" should be understood to encompass both endoscopic and laparoscopic procedures.

During a typical minimally invasive procedure, surgical objects, such as surgical access devices (e.g., trocar and cannula assemblies), or endoscopes, are inserted into the patient's body through the incision in tissue. In general, prior to the introduction of the surgical object into the patient's body, insufflation gas are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various ports with valves and seals are used during the course of minimally invasive procedures and are widely known in the art. However, a continuing need exists for a surgical portal that can be used with relative ease and with minor inconvenience for the surgeon.

### SUMMARY

Accordingly, a surgical portal includes a compressible portal having two or more longitudinal ports for passage of a surgical object. The portal is adapted to transition between a first condition having a first length and a second condition having a second length different from the first length. The portal includes an outer wall configured to maintain a substantial sealing relationship with tissue upon insertion therein. The outer wall and the one or more longitudinal ports truncate or elongate in response to the transition between the first condition and the second condition. In embodiments, the portal includes an insufflation passage disposed in mechanical cooperation with an insufflation conduit for the reception and passage of insufflation fluids. In embodiments, the portal is hour-glass shaped.

In one embodiment, a surgical portal includes a compressible, e.g., foam or rubber, portal having two or more longitudinal ports for passage of a surgical object that is adapted to be selectively positionable between a first condition having a first length and a second condition having a second length different from the first length. The portal includes an outer wall configured to maintain a substantial sealing relationship with tissue upon insertion therein. In this embodiment, the outer wall includes a plurality of segments, wherein each segment is movable with respect to the other segments upon the selective positioning between the first condition and the second condition. Each segment may be configured for uniform movement with respect to the other segments. Each segment expands outwardly in response to truncation of the portal and contracts inwardly in response to elongation of the portal.

In another embodiment, a surgical portal includes a compressible portal having two or more longitudinal ports for passage of a surgical object and includes a proximal member and a distal member. The portal is adapted to transition between a first condition having a first length and a second condition having a second length different from the first length. The distal member is configured to partially enclose an outer surface of the proximal member when the portal is disposed in the first condition. The proximal and distal members are configured to slidably engage. The distal member defines an extension bore and the proximal member includes an extension portion such that the extension portion is configured to engage the extension bore. The extension portion and the extension bore are slidably engaged. The extension portion is configured for a substantial sealing relationship with the extension bore and tissue. A portion of the extension portion may be offset from the distal member when the portal is disposed in the second condition.

### DESCRIPTION OF THE DRAWINGS

The above and other aspects, features, and advantages of the present disclosure will become more apparent in light of the following detailed description when taken in conjunction with the accompanying drawings in which:

FIG. 1A is a perspective view of one embodiment of a surgical portal in a first condition in accordance with the present disclosure;

FIG. 1B is a perspective view of the surgical portal of FIG. 1A in a second condition;

FIG. 2A is a perspective view of another embodiment of a surgical portal in a first condition in accordance with the present disclosure; and

FIG. 2B is a perspective view of the surgical portal of FIG. 2A in a second condition.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is farther from the user. In the following description, well-known functions or constructions are not described in detail to avoid obscuring the present disclosure in unnecessary detail.

One type of minimal invasive surgery described herein is referred to as a single-incision laparoscopic surgery (SILS). SILS is a minimally invasive surgical procedure, which permits a surgeon to operate through a single entry point, typically the patient's navel. The disclosed SILS procedure involves insufflating the body cavity and positioning a portal member within, e.g., the navel of the patient. Instruments including an endoscope and additional instruments such as graspers, staplers, forceps or the like may be introduced within the portal member to carry out the surgical procedure. An example of such a surgical portal is disclosed in commonly assigned U.S. patent application Serial No. 12/244,024, filed October 2, 2008, the entire content of which is hereby incorporated by reference herein.

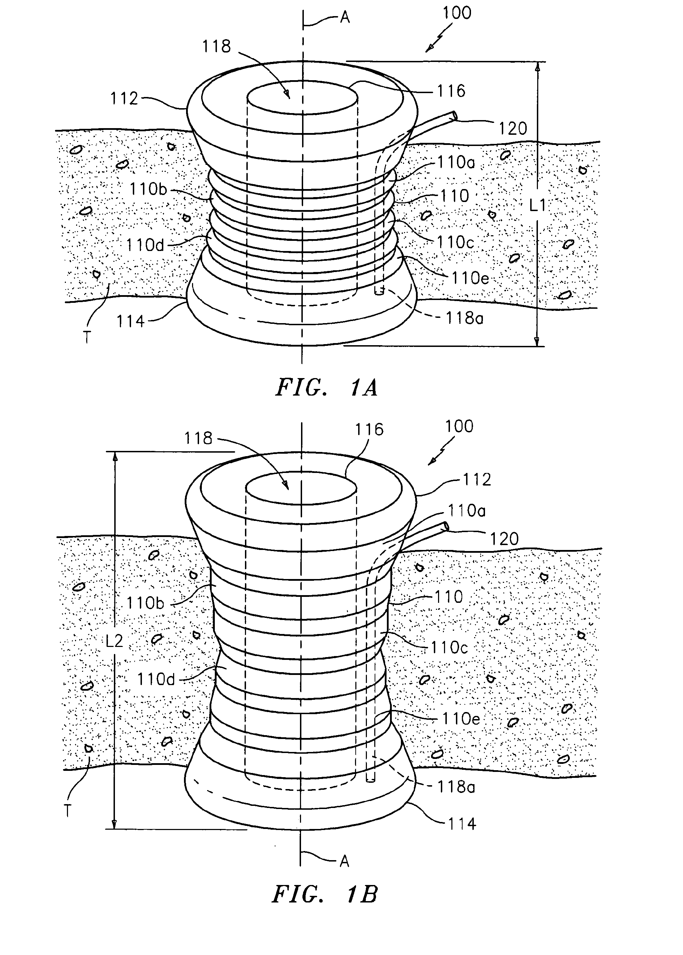

Referring now to the drawings, in which like reference numerals identify identical or substantially similar parts throughout the several views, FIGS. 1A and 1B illustrate a surgical portal 100 in accordance with the principles of the present disclosure.

Surgical portal 100 is adapted for insertion within a tissue tract "T", e.g., through the abdominal or peritoneal lining in connection with a laparoscopic surgical procedure. In particular, when inserted within the tissue tract "T", surgical portal 100 is adapted to establish a substantial seal within the tract "T", i.e., with the tissue surfaces defining the tract "T."

Surgical portal 100 includes an outer wall 110 configured to maintain a substantial sealing relationship with tissue "T" upon insertion therein. The outer wall 110 includes a plurality of segments 110a-110e, etc. Surgical portal 100 includes at least one longitudinal port 118, possibly, a plurality of longitudinal ports 118 extending along the longitudinal axis "L" thereof. The longitudinal ports 118 are dimensioned to receive, via insertion through a longitudinal opening 116, a surgical object, e.g. a surgical instrument (not shown) therethrough. Upon introduction of an instrument or surgical object (not shown) through a respective port 118, the inner surface portions defining the port 118 establish and maintain a substantial sealed relation about the instrument or surgical object.

Surgical portal 100 is adapted to transition between a first truncated condition (FIG. 1A) having a first length "L1" and a second elongated condition (FIG. 1B) having a second length "L2" longer than the first length "L1" in order to accommodate variously-sized tissue tracts "T." The outer wall 110 and the one or more longitudinal ports 118 truncate or elongate in response to the transition between the first truncated condition and the second elongated condition. Each segment of the plurality of segments 110a-110e of outer wall 110 is movable with respect to the other segments upon the selective positioning between the first truncated condition and the second elongated condition. Each segment may be configured for uniform movement with respect to the other segments. Each segment expands outwardly in response to truncation of the surgical portal 100 and contracts inwardly in response to elongation of the surgical portal 100.

Surgical portal 100 may define an hourglass shape as shown. Trailing and leading ends 112, 114 may define flange segments, which may be integrally formed with surgical portal 100. Surgical portal 100 may be made from a disposable, compressible, and/or flexible type material, for example, but not limited to, a suitable foam, gel material, or soft rubber having sufficient compliance to form a seal about one or more surgical objects, and also establish a sealing relation with tissue. The foam is preferably sufficiently compliant to accommodate off axis motion of the surgical object. In one embodiment, the foam includes a polyisoprene material.

During insertion, surgical portal 100 may be compressed to a compressed condition to permit at least partial passage through the tissue tract "T." Once within the tissue tract "T", surgical portal 100 will return toward the normal expanded condition with the outer wall 110 of the surgical portal 100 establishing a seal therewith. In particular, the leading or distal end 114 of the surgical portal 100 is positioned within the tissue tract "T" and the leading end 114 is advanced to a predetermined depth. Upon insertion, surgical portal 100 compresses to fit within the inner boundary the tissue tract "T." As the surgical portal 100 is advanced through the tissue tract "T", the surgical portal 100 expands toward its normal expanded condition in sealed engagement with tissue tract "T." Surgical portal 100 may be elongated or truncated prior to, during, or after insertion within the tissue tract "T" so that a physician may adjust the surgical portal 100 to accommodate variously-sized tissue tracts.

Surgical portal 100 may include an insufflation passage 118a disposed in mechanical cooperation with an insufflation conduit 120. The insufflation conduit 120 may be connectable to a source of insufflation fluid to permit passage of fluids (e.g., CO₂), to maintain the pneumoperitoneum.

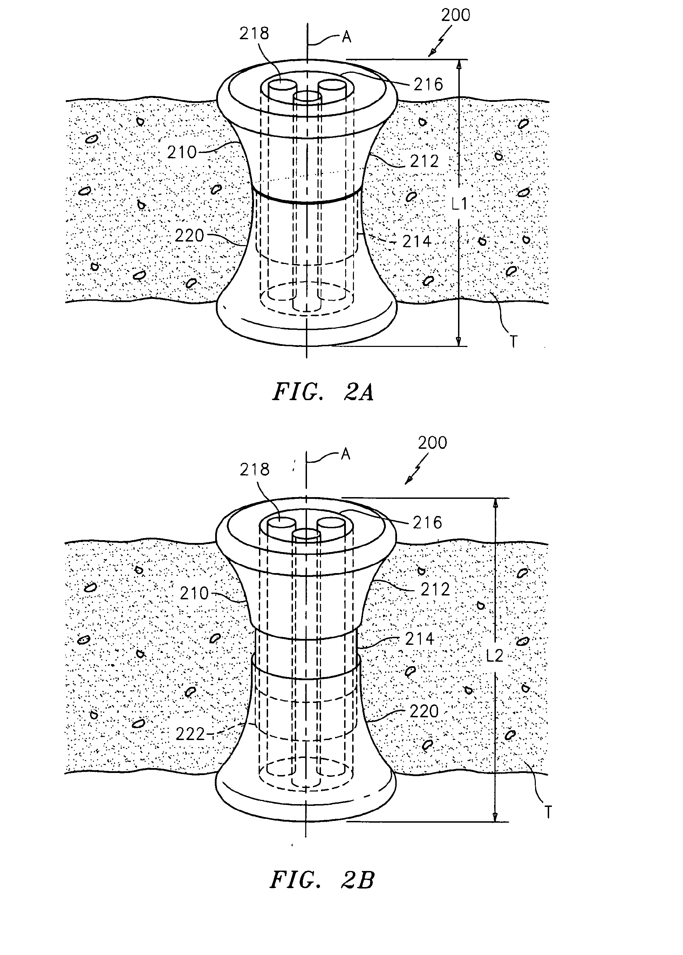

Referring now to FIGS. 2A and 2B, another embodiment of a surgical portal 200 includes a proximal member 210, a distal member 220, and one or more longitudinal ports 218 for passage of a surgical object therethrough. Surgical portal 200 is adapted to transition between a first truncated condition (FIG. 2A) having a first length "L1" and a second elongated condition (FIG. 2B) having a second length "L2" longer than the first length "L1." The distal member 220 is configured to at least partially enclose an outer surface 212 of the proximal member 210 when the surgical portal 200 is disposed in the first truncated condition. The proximal 210 and distal members 220 are configured to slidably engage. The distal member 220 defines an extension bore 222 and the proximal member 210 includes an extension portion 214 such that the extension portion 214 is configured to engage the extension bore 222. The extension portion 214 and the extension bore 222 are slidably engaged. The extension portion 214 is configured for a substantial sealing relationship with the extension bore 222 and tissue "T." At least a portion of the extension portion 214 may be offset from the distal member 220 when the surgical portal 200 is disposed in the second elongated condition.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The present invention may be described by reference to the following numbered paragraphs:-
1. A surgical portal, which comprises: a compressible portal having at least two longitudinal ports for passage of a surgical object, the portal being adapted to transition between a first condition having a first length and a second condition having a second length different from the first length, wherein the portal includes an outer wall configured to maintain a substantial sealing relationship with tissue upon insertion therein.
2. The surgical portal of paragraph 1, wherein the outer wall and the at least one longitudinal port truncates or elongates in response to the transition between the first condition and the second condition.
3. The surgical portal of paragraph 1, wherein the portal includes an insufflation passage disposed in mechanical cooperation with an insufflation conduit for the reception and passage of insufflation fluids.
4. The surgical portal of paragraph 1, wherein the portal includes three or more longitudinal ports.
5. The surgical portal of paragraph 1, wherein the portal is hour-glass shaped.
6. A surgical portal, which comprises: a compressible portal having at least two longitudinal ports for passage of a surgical object, the portal being adapted to be selectively positionable between a first condition having a first length and a second condition having a second length longer than the first length, wherein the portal includes an outer wall configured to maintain a substantial sealing relationship with tissue upon insertion therein, wherein the outer wall includes a plurality of segments, each segment being movable with respect to the other segments upon the selective positioning between the first condition and the second condition.
7. The surgical portal of paragraph 6, wherein each segment is configured for uniform movement with respect to the other segments.
8. The surgical portal of paragraph 6,wherein each segment expands outwardly in response to truncation of the portal and contracts inwardly in response to elongation of the portal.
9. A surgical portal, which comprises: a compressible portal having at least two longitudinal ports for passage of a surgical object and including a proximal member and a distal member, the portal being adapted to transition between a first condition having a first length and a second condition having a second length different from the first length, wherein the distal member is configured to at least partially enclose an outer surface of the proximal member when the portal is disposed in the first condition.
10. The surgical portal of paragraph 9, wherein the proximal and distal members are configured to slidably engage.
11. The surgical portal of paragraph 9, wherein the distal member defines an extension bore and the proximal member includes an extension portion such that the extension portion is configured to engage the extension bore.
12. The surgical portal of paragraph 11, wherein the extension portion and the extension bore are slidably engaged.
13. The surgical portal of paragraph 11, wherein the extension portion is configured for a substantial sealing relationship with the extension bore and tissue.
14. The surgical portal of paragraph 11, wherein at least a portion of the extension portion is offset from the distal member when the portal is disposed in the second condition.

## Claims

1. A surgical portal, which comprises:
a compressible portal having at least two longitudinal ports for passage of a surgical object, the portal being adapted to transition between a first condition having a first length and a second condition having a second length different from the first length, wherein the portal includes an outer wall configured to maintain a substantial sealing relationship with tissue upon insertion therein.

2. The surgical portal of claim 1, wherein the outer wall and the at least one longitudinal port truncates or elongates in response to the transition between the first condition and the second condition.

3. The surgical portal of claim 1 or claim 2, wherein the portal includes an insufflation passage disposed in mechanical cooperation with an insufflation conduit for the reception and passage of insufflation fluids.

4. The surgical portal of any preceding claim, wherein the portal includes three or more longitudinal ports.

5. The surgical portal of any preceding claim, wherein the portal is hour-glass shape.

6. A surgical portal, which comprises:
a compressible portal having at least two longitudinal ports for passage of a surgical object, the portal being adapted to be selectively positionable between a first condition having a first length and a second condition having a second length longer than the first length, wherein the portal includes an outer wall configured to maintain a substantial sealing relationship with tissue upon insertion therein, wherein the outer wall includes a plurality of segments, each segment being movable with respect to the other segments upon the selective positioning between the first condition and the second condition.

7. The surgical portal of claim 6, wherein each segment is configured for uniform movement with respect to the other segments.

8. The surgical portal of claim 6 or claim 7,wherein each segment expands outwardly in response to truncation of the portal and contracts inwardly in response to elongation of the portal.

9. A surgical portal, which comprises:
a compressible portal having at least two longitudinal ports for passage of a surgical object and including a proximal member and a distal member, the portal being adapted to transition between a first condition having a first length and a second condition having a second length different from the first length, wherein the distal member is configured to at least partially enclose an outer surface of the proximal member when the portal is disposed in the first condition.

10. The surgical portal of claim 9, wherein the proximal and distal members are configured to slidably engage.

11. The surgical portal of claim 9 or claim 10, wherein the distal member defines an extension bore and the proximal member includes an extension portion such that the extension portion is configured to engage the extension bore.

12. The surgical portal of claim 11, wherein the extension portion and the extension bore are slidably engaged.

13. The surgical portal of claim 11 or claim 12, wherein the extension portion is configured for a substantial sealing relationship with the extension bore and tissue.

14. The surgical portal of any of claims 11 to 13, wherein at least a portion of the extension portion is offset from the distal member when the portal is disposed in the second condition.
